(19) Europäisches Patentamt
European Patent Office
Office européen des brevets

(11) EP 0 566 919 B1

(12) **EUROPÄISCHE PATENTSCHRIFT**

(45) Veröffentlichungstag und Bekanntmachung des Hinweises auf die Patenterteilung:
**09.04.2003 Patentblatt 2003/15**

(51) Int Cl.$^7$: **C07D 233/96**, C07D 233/76, C08K 5/08, A61K 38/06, A61P 7/02

(21) Anmeldenummer: **93105575.0**

(22) Anmeldetag: **03.04.1993**

(54) **2,4-Dioxo-imidazolidin-Derivate**

2,4-dioxoimidazoline derivatives

Dérivés de 2,4-dioxoimidazoline

(84) Benannte Vertragsstaaten:
**AT BE CH DE DK ES FR GB IT LI NL SE**

(30) Priorität: **24.04.1992 DE 4213634**

(43) Veröffentlichungstag der Anmeldung:
**27.10.1993 Patentblatt 1993/43**

(73) Patentinhaber: **HOECHST AKTIENGESELLSCHAFT**
**65926 Frankfurt am Main (DE)**

(72) Erfinder:
- **Zoller, Gerhard, Dr.**
  **W-6369 Schöneck (DE)**
- **Just, Melitta, Dr.**
  **W-6070 Langen (DE)**
- **Jablonka, Bernd, Dr.**
  **W-6232 Bad Soden (DE)**
- **König, Wolfgang, Dr.**
  **W-6238 Hofheim (DE)**
- **Knolle, Jochen, Dr.**
  **W-6239 Kriftel (DE)**

(56) Entgegenhaltungen:
**EP-A- 0 449 079** **DE-A- 4 126 277**
**US-A- 3 300 510**

- **JOURNAL OF THE CHEMICAL SOCIETY, Dezember 1965, LONDON, GB Seiten 6806 - 6813 M.E. COX ET AL. 'Amino-acids and Peptides. Part XXIII. The Synthesis of Peptides Related to Arginine-vasopressin'**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die vorliegende Erfindung betrifft 2,4-Dioxo-imidazolidin-Derivate, ihre Herstellung und ihre Verwendung als Hemmstoffe der Blutplättchenaggregation.

In der US-A-3 300 510 sind bestimmte N-(α,α-Dimethylphenethyl)-hydantoinacetamide beschrieben, die unter anderem eine antidepressive Wirkung zeigen. In der EP-A-449 079 sowie in der DE-A-41 26 277 sind Hydantoinderivate mit thrombozytenaggregationshemmender Wirkung beschrieben. Weitere Forschungsarbeiten haben gezeigt, daß auch die Verbindungen der vorliegenden Erfindung starke Hemmstoffe der Blutplättchenaggregation sind.

Die vorliegende Erfindung betrifft Verbindungen der allgemeinen Formel I

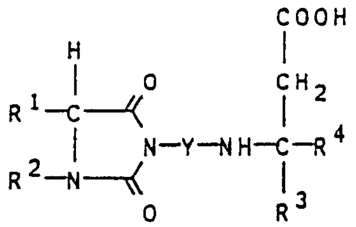

(I)

worin

Y $-(CH_2)_m-CO-$, wobei m für 1 oder 2 steht, oder

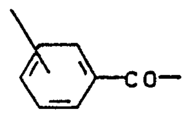

bedeutet;

$R^1$ $-CH_2-C_6H_4-NH-C(=NH)-NH_2$, $-CH_2-C_6H_4-C(=NH)-NH_2$ oder $-CH_2-C_6H_4-CH_2-NH_2$ bedeutet;

$R^2$ Wasserstoff oder Methyl bedeutet;

$R^3$ Wasserstoff bedeutet; und

$R^4$ $-CO-NH-R^5$ bedeutet, wobei $-NH-R^5$ für ein ω-Amino-($C_2$-$C_8$)-alkylamid des Valin-, Lysin-, Phenylalaninoder des Phenylglycinrestes steht;

sowie deren physiologisch verträgliche Salze.

Alkylreste können geradkettig oder verzweigt sein. Beispiele für Alkylreste sind Methyl, Ethyl, n-Propyl, i-Propyl, n-Butyl, i-Butyl, sek-Butyl und tert.-Butyl. Entsprechendes gilt für Reste wie Alkoxy oder Alkoxycarbonyl.

Natürliche und unnatürliche Aminosäuren können in der D- oder L-Form vorliegen.

Physiologisch verträgliche Salze der Verbindungen der allgemeinen Formel I sind insbesondere pharmazeutisch verwendbare oder nicht-toxische Salze.

Solche Salze werden beispielsweise von Verbindungen der allgemeinen Formel I, welche saure Gruppen, z. B. Carboxy, enthalten, mit Alkali- oder Erdalkalimetallen gebildet, wie z. B. Na, K, Mg und Ca, sowie mit physiologisch verträglichen organischen Aminen, wie z. B. Triethylamin und Tris-(2-hydroxy-ethyl)-amin.

Verbindungen der allgemeinen Formel I, welche basische Gruppen, z. B. eine Aminogruppe oder eine Guanidinogruppe enthalten, bilden mit anorganischen Säuren, wie z. B. Salzsäure, Schwefelsäure oder Phosphorsäure und mit organischen Carbon- oder Sulfonsäuren, wie z. B. Essigsäure, Citronensäure, Benzoesäure, Maleinsäure, Fumarsäure, Weinsäure und p-Toluolsulfonsäure Salze.

Für $-NH-R^5$ steht bevorzugt das 4-Aminobutylamid des Valin-, Lysin-, Phenylalanin- oder des Phenylglycin-Restes.

Die erfindungsgemäßen Verbindungen der allgemeinen Formel I können hergestellt werden durch Fragmentkondensation einer Verbindung der allgemeinen Formel III

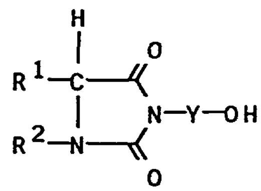

(III)

mit einer Verbindung der allgemeinen Formel IV

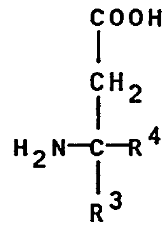

(IV)

wobei die Reste $R^1$ bis $R^4$ und Y wie oben angegeben definiert sind.

Zur Kondensation der Verbindungen der allgemeinen Formel III mit denen der allgemeinen Formel IV verwendet man vorteilhafterweise die an sich bekannten Methoden der Peptidchemie (siehe z. B. Houben-Weyl, Methoden der organischen Chemie, Band 15/1 und 15/2, Stuttgart, 1974).

Dazu ist es in der Regel nötig, daß in $R^1$ und $R^4$ enthaltene Aminogruppen durch reversible Schutzgruppen geschützt werden. Gleiches gilt für die Carboxylgruppen der Verbindung der allgemeinen Formel IV, die bevorzugt als Benzyl- oder tert.-Butylester vorliegen. Ein Aminogruppen-Schutz erübrigt sich, wenn die zu generierenden Aminogruppen als Nitro- oder Cyanogruppen vorliegen und erst nach der Kupplung durch Hydrierung gebildet werden.

Nach der Kupplung werden die vorhandenen Schutzgruppen in geeigneter Weise abgespalten. Beispielsweise können $NO_2$-Gruppen (Guanidinoschutz), Benzyloxycarbonylgruppen und Benzylester abhydriert werden. Schutzgruppen vom tert.-Butyltyp werden sauer gespalten, während der 9-Fluorenylmethyloxycarbonylrest durch sekundäre Amine entfernt wird.

Die Ausgangsverbindungen der allgemeinen Formel III können wie folgt erhalten werden:

Durch Umsetzung von Aminosäuren, N-Alkylaminosäuren oder bevorzugt deren Methyl-, Ethyl-, Benzyl- oder tert.-Butylester, beispielsweise eine Verbindung der allgemeinen Formel V

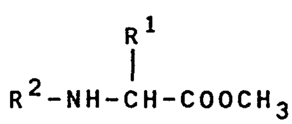

(V)

mit einem Isocyanatoalkancarbonsäureester, beispielsweise der allgemeinen Formel VI

$$O{=}C{=}N\text{-}(CH_2)_m\text{-}COOCH_3 \qquad \text{(VI)}$$

worin $R^1$, $R^2$ und m wie oben angegeben definiert sind, erhält man Harnstoffderivate, beispielsweise der allgemeinen Formel VII

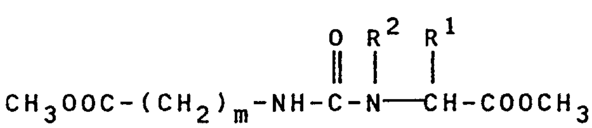

(VII),

die durch Erhitzen mit Säure unter Verseifung der Esterfunktionen zu Verbindungen der allgemeinen Formel IIIa

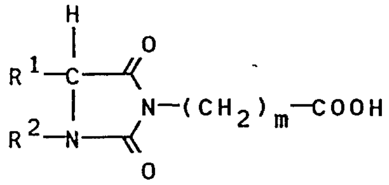

(IIIa)

cyclisieren.

Während der Harnstoffsynthese können Guanidinogruppen durch Schutzgruppen, wie $NO_2$ oder Mtr, blockiert werden. Ebenso müssen Aminogruppen in der Seitenkette in geschützter Form (beispielsweise als Boc- oder Z-Derivate) oder noch als $NO_2$- oder Cyanofunktion vorliegen, die später zur Aminogruppe reduziert oder im Falle der Cyanogruppe auch in die Formamidinogruppe umgewandelt werden kann.

Verbindungen der allgemeinen Formel IIIb

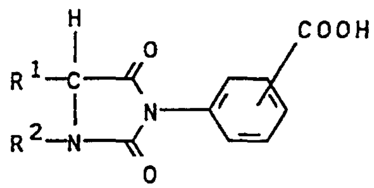

(IIIb)

können analog erhalten werden, wenn anstelle von Isocyanatoalkancarbonsäureestern die Isocyanate der Aminobenzoesäureester eingesetzt werden.

Im übrigen entstehen Hydantoine der allgemeinen Formel Xa

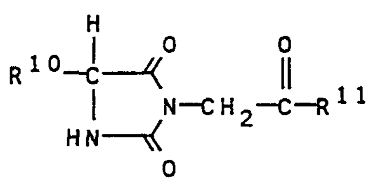

(Xa)

worin $R^{10}$ eine beliebige Aminosäureseitenkette und $R^{11}$ ein Amid, einen Aminosäure- oder einen Peptidrest bedeuten, ganz allgemein durch basische Behandlung von Alkyloxycarbonyl- oder Aralkyloxycarbonylpeptiden der allgemeinen Formel X

$$R^{12}\text{-O-CO-NH-CHR}^{10}\text{-CO-NH-CH}_2\text{-CO-R}^{11} \qquad (X)$$

worin $R^{10}$ und $R^{11}$ wie oben angegeben definiert sind und $R^{12}$ Benzyl oder tert.-Butyl bedeutet (J. S. Fruton und M. Bergmann, J. Biol. Chem. 145 (1942) 253 - 265; C. A. Dekker, S. P. Taylor, jr. und J. S. Fruton, J. Biol. Chem. 180 (1949) 155 - 173; M. E. Cox, H. G. Carg, J. Hollowood, J. M. Hugo, P. M. Scopes und G. T. Young, J. Chem. Soc. (1965) 6806 - 6813; W. Voelter und A. Altenburg, Liebigs Ann. Chem. (1983) 1641 - 1655;
B. Schwenzer, E. Weber und G. Losse, J. Prakt. Chem. 327 (1985) 479 - 486). Dabei razemisiert jedoch die N-terminale Aminosäure und das Hydantoin hydrolysiert in das Harnstoffderivat

$$\text{HOCO-CHR}^{10}\text{-NH-CO-NH-CH}_2\text{-CO-R}^{11}$$

(W. Voelter und A. Altenburg, Liebigs Ann. Chem. (1983) 1641 - 1655).

Eine milde Methode ist dagegen die Zyklisierung zu den Hydantoinen aus Verbindungen der allgemeinen

Formel X durch Behandlung mit Tetrabutylammoniumfluorid in Tetrahydrofuran unter Rückfluß (J. Pless, J. Org. Chem. 39 (1974) 2644 - 2646).

Eine weitere Möglichkeit einer milden Zyklisierung ist die Trimethylsilylierung der Peptidbindung zwischen der N-terminalen Aminosäure und dem folgenden Glycin mit Bistrimethylsilyltrifluoracetamid in Acetonitril (4 Stunden unter Rückfluß) (J. S. Davies, R. K. Merritt und R. C. Treadgold, J. Chem. Soc. Perkin Trans. I (1982) 2939 - 2947).

Die Guanylierung der Aminofunktion kann mit folgenden Reagentien durchgeführt werden:

1. O-Methylisothioharnstoff (S. Weiss und H. Krommer, Chemiker Zeitung 98 (1974) 617 - 618),

2. S-Methylisothioharnstoff (R. F. Borne, M. L. Forrester und I. W. Waters, J. Med. Chem. 20 (1977) 771 - 776),

3. Nitro-S-Methylisothioharnstoff (L. S. Hafner und R. E. Evans, J. Org. Chem. 24 (1959) 1157),

4. Formamidinosulfonsäure (K. Kim, Y.-T. Lin und H. S. Mosher, Tetrah. Lett. 29 (1988) 3183 - 3186),

5. 3,5-Dimethyl-1-pyrazolyl-formamidinium-nitrat (F. L. Scott, D. G. O'Donovan und J. Reilly, J. Amer. Chem. Soc. 75 (1953) 4053 - 4054).

Formamidine können aus den entsprechenden Cyanoverbindungen durch Anlagerung von Alkoholen (z. B. Methanol oder Ethanol) in saurem wasserfreiem Medium (z. B. Dioxan, Methanol oder Ethanol) und anschließender Behandlung mit Ammoniak in Alkoholen (z. B. Isopropanol, Methanol oder Ethanol) hergestellt werden (G. Wagner, P. Richter und Ch. Garbe, Pharmazie 29 (1974) 12 - 55). Eine weitere Methode, Formamidine herzustellen, ist die Anlagerung von $H_2S$ an die Cyanogruppe, gefolgt von einer Methylierung des entstandenen Thioamids und anschließender Umsetzung mit Ammoniak (DDR-Patent Nr. 235 866).

Die Ausgangspeptide der allgemeinen Formel IV werden in der Regel vom C-terminalen Ende her stufenweise aufgebaut. Peptidknüpfungen können mit den bekannten Kupplungsmethoden der Peptidchemie durchgeführt werden.

Die Verbindungen der allgemeinen Formel I und ihre physiologisch verträglichen Salze können als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Salzes davon, neben üblichen pharmazeutisch einwandfreien Träger- und Zusatzstoffen enthalten. Die Zubereitungen enthalten normalerweise etwa 0,5 bis 90 Gew% der therapeutisch wirksamen Verbindung.

Die Heilmittel können oral, z. B. in Form von Pillen, Tabletten, Lacktabletten, Dragees, Granulaten, Hart- und Weichgelatinekapseln, Lösungen, Sirupen, Emulsion oder Suspensionen oder Aerosolmischungen verabreicht werden. Die Verabreichung kann aber auch rektal, z. B. in Form von Suppositorien oder parenteral, z. B. in Form von Injektionslösungen oder Mikrokapseln, perkutan, z. B. in Form von Salben oder Tinkturen, oder nasal, z. B. in Form von Nasalsprays, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z. B. Lactose, Maisstärke oder Derivate davon, Talk, Stearinsäure oder deren Salze etc. verwenden. Trägerstoffe für Weichgelatinekapseln und Suppositorien sind z. B. Fette, Wachse, halbfeste und flüssige Polyole, natürliche oder gehärtete Öle etc. Als Trägerstoffe für die Herstellung von Lösungen und Sirupen eignen sich z. B. Wasser, Saccharose, Invertzucker, Glukose, Polyole etc. Als Trägerstoffe für die Herstellung von Injektionslösungen eignen sich Wasser, Alkohole, Glycerin, Polyole, pflanzliche Öle etc. Als Trägerstoffe für Mikrokapseln oder Implantate eignen sich Mischpolymerisate aus Glykolsäure und Milchsäure.

Die pharmazeutischen Präparate können neben den Wirk- und Trägerstoffen noch Zusatzstoffe, wie z. B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten.

Sie können auch zwei oder mehrere Verbindungen der allgemeinen Formel I oder ihrer physiologisch verträglichen Salze und noch einen oder mehrere andere therapeutisch wirksame Stoffe enthalten.

Derartige andere therapeutisch wirksame Substanzen sind beispielsweise durchblutungsfördernde Mittel, wie Dihydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Verbindungen, wie Digoxin, Acetyldigoxin, Metildigoxin und Lantano-Glykoside; Coronardilatatoren, wie Carbochromen; Dipyridamol, Nifedipin und Perhexilin; antianginöse Verbindungen, wie Isosorbiddinitrat, Isosorbidmononitrat, Glycerolnitrat, Molsidomin und Verapamil; β-Blocker, wie Propranolol, Oxprenolol, Atenolol, Metoprolol und Penbutolol. Darüberhinaus lassen sich die Verbindungen mit anderen nootrop wirksamen Substanzen, wie z. B. Piracetam, oder ZNS-aktiven Substanzen, wie Pirlindol, Sulpirid etc., kombinieren.

Die Dosis kann innerhalb weiter Grenzen variieren und ist in jedem einzelnen Fall den individuellen Gegebenheiten anzupassen. Im allgemeinen ist bei der oralen Verabreichung eine Tagesdosis von etwa 0,1 bis 1 mg/kg, vorzugsweise 0,3 bis 0,5 mg/kg Körpergewicht zur Erzielung wirksamer Ergebnisse angemessen, bei intravenöser Applikation beträgt die Tagesdosis im allgemeinen etwa 0,01 bis 0,3 mg/kg, vorzugsweise 0,05 bis 0,1 mg/kg Körpergewicht. Die Tagesdosis wird normalerweise, insbesondere bei der Applikation größerer Mengen, in mehrere, z. B. 2, 3 oder 4 Teilverabreichungen aufgeteilt. Gegebenenfalls kann es, je nach individuellem Verhalten, erforderlich werden, von der angegebenen Tagesdosis nach oben oder nach unten abzuweichen. Pharmazeutische Präparate enthalten normalerweise 0,2 bis 50 mg, vorzugsweise 0,5 bis 10 mg Wirkstoff der allgemeinen Formel I oder eines ihrer physiologisch verträglichen Salze pro Dosis.

Die erfindungsgemäßen Verbindungen der Formel I haben die Fähigkeit die Zell-Zell-Adhäsion zu hemmen, die auf der Interaktion von Arg-Gly-Asp-enthaltenden Proteinen, wie Fibronectin, Fibrinogen oder des von Willebrand-Faktors mit den sogenannten Integrinen beruhen. Integrine sind Transmembran-Glykoproteine, Rezeptoren für Arg-Gly-Aspenthaltende Zellmatrix-Glykoproteine (E. Ruoslahti und M. D. Pierschbacher, Science 238 (1987) 491 - 497; D. R. Phillips, I. F. Charo, L. V. Parise und L. A. Fitzgerald, Blood 71 (1988) 831 - 843). Außerdem hemmen sie die Bindung weiterer adhäsiver Proteine, wie Vitronectin, Kollagen und Laminin an die entsprechenden Rezeptoren auf der Oberfläche verschiedener Zelltypen.

Die erfindungsgemäßen Verbindungen der allgemeinen Formel I hemmen die Thrombozytenaggregation, die Metastasierung von Karzinomzellen sowie die Osteoclastenbindung an die Knochenoberflächen.

Die Hydantoinderivate der allgemeinen Formel I finden akut Anwendung bei Thrombosegefahr und chronisch bei der Prävention der Arteriosklerose und Thrombose, z. B. bei der Therapie und Prophylaxe arterieller Gefäßerkrankungen, wie bei akutem Myokardinfarkt, Sekundärprävention des Myokardinfarkts, Reokklusionsprophylaxe nach Lyse und Dilatation (PTCA), instabiler Angina pectoris, transitorischen ischämischen Attacken, Schlaganfall, koronarer Bypass-Operation einschließlich Reokklusionsprophylaxe bei Bypass, Lungenembolie, peripherer arterieller Verschlußkrankheit, Dissezierendem Aneurysma; bei der Therapie venöser und mikrozirkulatorischer Gefäßerkrankungen, wie tiefer Venenthrombose, disseminenter intravaskulärer Gerinnung, postoperativem und post-partum Trauma, chirurgischem oder infektiösem Schock, Septicämie oder bei Erkrankungen mit hyperreagiblen Thrombozyten, thrombotischer thrombozytopenischer Purpura, Preeklampsie, prämenstruellem Syndrom, Dialyse oder extrakorporaler Zirkulation; eine weitere Anwendung ist während Krebsoperationen und auch prophylaktisch bei Krebs gegeben. Ferner kann Osteoporose durch Hemmung der Osteociastenbindung an die Knochenoberfläche verhindert werden.

Geprüft werden die Verbindungen vor allem auf ihre hemmende Wirkung bei der Blutplättchenaggregation und der Anhaftung von Fibrinogen an Blutplättchen. Verwendet werden gelfiltrierte Blutplättchen aus humanem Spenderblut, die mit ADP oder Thrombin aktiviert werden.

## Beispiele:

Die Produkte wurden über Massenspektren und/oder NMR-Spektren identifiziert.

### Beispiel 1: Illustrierung der Herstellmethode

**(5-(S)-(3-Guanidinopropyl)-2,4-dioxo-imidazolidin-3-yl)-acetyl-L-aspartyl-L-phenylalanin-(4-aminobutyl)-amidacetat**

### 1a: Z-Phe-NH-$(CH_2)_4$-NH-Boc

5,98 g (20 mmol) N-Benzyloxycarbonyl-L-Phenylalanin und 4,49 g (20 mmol) 4-(tert.-Butyloxycarbonylamino)-butylamin-hydrochlorid werden in 100 ml Dimethylformamid gelöst und im Eisbad auf 0°C abgekühlt. Nach Zugabe von 2,7 g (20 mmol) Hydroxybenzotriazol, 4,4 g (20 mmol) DCCI und 2,54 ml (20 mmol) N-Ethylmorpholin wird die Reaktion über Nacht gerührt. Der ausgefallene Harnstoff wird abgesaugt und die Lösung wird im Hochvakuum eingedampft. Der Rückstand wird in 200 ml Essigsäureethylester aufgenommen und die organische Phase wird mit Wasser, Natriumhydrogencarbonatlösung und Kaliumhydrogensulfatlösung extrahiert und über Magnesiumsulfat getrocknet. Nach Filtration und Einengen erhält man 8,2 g (87 %) amorphes Produkt.

### 1b: H-Phe-NH-$(CH_2)_4$-NH-Boc-hydrochlorid

8,2 g (17,5 mmol) Z-Phe-NH-$(CH_2)_4$-NH-Boc werden in 300 ml Methanol gelöst und mit 1,5 g Pd/C versetzt. Anschließend leitet man Wasserstoff ein und hält den pH-Wert durch Zugabe von methanolischer Salzsäure auf pH 4. Nach Beendigung der Reaktion wird filtriert und das Filtrat eingedampft. Nach Verreiben mit Ether erhält man 6,5 g

(100 %) amorphes Produkt.

### 1c: H-L-Aspartyl(OtBu)-L-phenylalanin-NH-$(CH_2)_4$-NH-Bochydrochlorid

5,6 g (17,4 mmol) Z-Asp(OtBu)-OH und 6,5 g (17,5 mmol) H-Phe-NH-$(CH_2)_4$-NH-Boc-hydrochlorid werden in 100 ml Dimethylformamid gelöst. Nach Zugabe von 2,3 g (17 mmol) Hydroxybenzotriazol, 4,4 g (21,3 mmol) DCCI und 2,2 ml (17,3 mmol) N-Ethylmorpholin wird die Reaktion über Nacht gerührt. Nach Beendigung der Reaktion engt man im Vakuum ein. Der Rückstand wird in Essigsäureethylester aufgenommen und die organische Phase wird mit Wasser und Natriumhydrogencarbonatlösung extrahiert und anschließend mit Magnesiumsulfat getrocknet. Nach Filtration und Einengen erhält man 9,7 g Rohprodukt, welches durch Chromatographie gereinigt wird.

Die vereinigten Fraktionen werden, wie unter 1b beschrieben, hydriert. Der Rückstand der Hydrierung wird in 50 ml Essigsäureethylester gelöst und mit Petrolether präzipitiert. Man erhält 4,9 g.
FAB-MS 507,3 $(M+H)^+$

### 1d: 5-(S)-(3-Guanidinopropyl)-2,4-dioxo-imidazolidin-3-yl)-acetyl-L-aspartyl(OtBu)-L-phenylalanin-(4-Boc-aminobutyl)-amid

977 mg (1,8 mmol) H-L-Aspartyl(OtBu)-L-phenylalanin-NH-$(CH_2)_4$-NH-Boc-hydrochlorid und 500 mg 5-(S)-(3-Guanidinopropyl)-2,4-dioxo-imidazolidin-3-yl)-essigsäure werden in 5 ml Dimethylformamid gelöst. Zur Lösung werden 243 mg (1,8 mmol) Hydroxybenzotriazol und 412 mg (2 mmol) DCCI hinzugegeben und über Nacht gerührt. Nach Beendigung der Reaktion wird die Lösung eingeengt und der Rückstand ohne weitere Behandlung an Kieselgel chromatographiert (Laufmittel: $CH_2Cl_2$, $CH_3OH$, Essigsäure, Wasser = 85 : 10 : 2,5 : 2,5). Es werden 1,03 g (76 %) Produkt isoliert.
FAB-MS 746,6 $(M+H)^+$

### 1e: (5-(S)-(3-Guanidinopropyl)-2,4-dioxo-imidazolidin-3-yl)-acetyl-L-aspartyl-L-phenylalanin-(4-aminobutyl)-amidacetat

1 g (1,38 mmol) 5-(S)-(3-Guanidinopropyl)-2,4-dioxo-imidazolidin-3-yl)-acetyl-L-aspartyl(OtBu)-L-phenylalanin-(4-Boc-aminobutyl)-amid werden in 15 ml 90 %iger wäßriger Trifluoressigsäure 1 Stunde bei Raumtemperatur gerührt. Anschließend wird die Lösung eingeengt, der Rückstand in Wasser aufgenommen und mit Ionenaustauscher IRA-93 behandelt, bis ein pH-Wert von 4 erreicht wird. Die Lösung wird gefriergetrocknet und der Rückstand (880 mg) mit 1 m Essigsäure an Sephadex LH20 chromatographiert. Nach Einengen und Gefriertrocknung erhält man 735 mg (90 %) Produkt.
FAB-MS 590,1 $(M+H)^+$

### Beispiel 4:

### (5-(R,S)-(4-Formamidinobenzyl)-2,4-dioxo-imidazolidin-3-yl)-acetyl-L-aspartyl-L-phenylalanin-(4-aminobutyl)-amidacetat

Diese Verbindung wurde analog der in Beispiel 1 beschriebenen Methode hergestellt.
FAB-MS 623 $(M+H)^+$

### Beispiel 5:

### (5-(R,S)-(4-Formamidinobenzyl)-2,4-dioxo-imidazolidin-3-yl)-acetyl-L-aspartyl-L-phenylalanin-(6-aminohexyl)-amidacetat

Diese Verbindung wurde analog der in Beispiel 1 beschriebenen Methode hergestellt.
FAB-MS 651 $(M+H)^+$

### Beispiel 6:

### (5-(R,S)-(4-Formamidinobenzyl)-2,4-dioxo-imidazolidin-3-yl)-acetyl-L-aspartyl-L-phenylalanin-(8-aminooctyl)-amidacetat

Diese Verbindung wurde analog der in Beispiel 1 beschriebenen Methode hergestellt.

FAB-MS 679 $(M+H)^+$

**Beispiel 7:**

**(5-(R,S)-(4-Formamidino-benzyl)-2,4-dioxo-imidazolidin-3-yl)-acetyl-L-aspartyl-L-lysin-(4-aminobutyl)-amid**

**7a: H-Aspartyl(OtBu)-L-lysin(Boc)-NH-$(CH_2)_4$-NH-Boc-hydrochlorid**

Zu einer Lösung von 5,3 g H-Lys(Boc)-NH-$(CH_2)_4$-NH-Boctosylat, 2,91 g Z-Asp(OtBu)-OH, 1,21 g Hydroxybenzotriazol in 20 ml Dimethylformamid gibt man bei 0 °C 1,17 ml N-Ethylmorpholin und 1,98 g DCCI. Man rührt 1 Stunde bei 0 °C und 4 Stunden bei Raumtemperatur und läßt über Nacht bei Raumtemperatur stehen. Der Niederschlag wird abgesaugt und das Filtrat im Vakuum eingeengt. Der Rückstand wird zwischen Essigsäureethylester und Wasser verteilt. Die organische Phase wird mit Natriumhydrogencarbonatlösung, Kaliumhydrogensulfatlösung und Wasser extrahiert und über Natriumsulfat getrocknet und eingeengt. Der Rückstand wird mit Ether verrieben und abgesaugt. Das erhaltene Produkt (5,2 g) wird in 150 ml Methanol gelöst und an einer Autobürette mit methanolischer Salzsäure bei pH 4,5 über Pd/Kohle katalytisch hydriert. Nach beendeter Reaktion wurde der Katalysator abgesaugt und das Filtrat eingeengt.
Ausbeute: 4,12 g amorphe Substanz
$\alpha)_D^{24}$ = + 3,9° (c = 1, Methanol)

**7b:(5-(R,S)-(4-Formamidinobenzyl)-2,4-dioxo-imidazolidin-3-yl)-acetyl-L-aspartyl(OtBu)-L-lysin(Boc)-(4-Bocaminobutyl)-amid**

Zu einer Suspension von 1,54 g (5-(R,S)-(4-Formamidinobenzyl)-2,4-dioxo-imidazolidin-3-yl)-essigsäure, 3,1 g H-Aspartyl(OtBu)-L-lysin(Boc)-NH-$(CH_2)_4$-NH-Boc-hydrochlorid und 675 mg Hydroxybenzotriazol in 20 ml Dimethylformamid gibt man bei 0 °C 1,1 g DCCI. Man rührt 1 Stunde bei 0 °C und 4 Stunden bei Raumtemperatur und läßt über Nacht bei Raumtemperatur stehen. Der Niederschlag wird abgesaugt und das Filtrat im Vakuum eingeengt. Zur Reinigung wird die Substanz über Kieselgel in Methylenchlorid/Methanol/Wasser/Essigsäure = 8,5 : 1,5 : 0,2 : 0,2 chromatographiert.
Ausbeute: 3,26 g amorphe Substanz
$\alpha)_D^{24}$ = - 28,7° (c = 1, Methanol)

**7c: (5-(R,S)-(4-Formamidinobenzyl)-2,4-dioxo-imidazolidin-3-yl)-acetyl-L-aspartyl-L-lysin-(4-aminobutyl)-amiddiacetat**

3,15 g (5-(R,S)-(4-Formamidinobenzyl)-2,4-dioxo-imidazolidin-3-yl)-acetyl-L-aspartyl(OtBu)-L-lysin(Boc)-(4-Bocaminobutyl)-amid werden in 30 ml einer 90 %igen wässrigen Trifluoressigsäure gelöst. Nach einer Stunde bei Raumtemperatur wird im Vakuum eingeengt und der Rückstand zwischen Wasser und Diethylether verteilt. Die wässrige Phase (30 ml) wird über 50 ml Amberlite IR 93 (Acetat-Form) und Wasser als Laufmittel chromatographiert. Das Eluat wird gefriergetrocknet und ergibt 2,42 g Substanz. Zur Reinigung wird die Substanz an Sephadex LH20 (200 x 4 cm) in einer Mischung aus Essigsäure, n-Butanol und Wasser chromatographiert. Die Fraktionen mit reiner Substanz werden eingeengt, in Wasser gelöst und gefriergetrocknet.
Ausbeute 2,27 g
$\alpha)_D^{24}$ = - 35,2° (c = 1, Wasser)

**Beispiel 9:**

**(5-(R,S)-(4-Formamidinobenzyl)-2,4-dioxo-imidazolidin-3-yl)-acetyl-L-aspartyl-L-phenylglycin-(4-aminobutyl)-amid**

Diese Verbindung wurde analog der in Beispiel 1 beschriebenen Methode hergestellt.
FAB-MS 608 $(M+H)^+$

**Beispiel A:**

Emulsionen mit 3 mg Wirkstoff per 5 ml können nach folgender Rezeptur hergestellt werden:

| | |
|---|---|
| Wirkstoff | 0,06 g |
| Neutralöl | q. s. |
| Natriumcarboxymethylzellulose | 0,6 g |
| Polyoxyethylenstearat | q. s. |
| Reinglycerin | 0,6 bis 2 g |
| Aromastoffe | q. s. |
| Wasser (entmineralisiert oder destilliert) | ad 100 ml |

**Beispiel B:**

Tabletten können nach folgender Formulierung hergestellt werden:

| | |
|---|---|
| Wirkstoff | 2 mg |
| Lactose | 60 mg |
| Maisstärke | 30 mg |
| lösliche Stärke | 4 mg |
| Magnesiumstearat | 4 mg |
| | 100 mg |

**Beispiel C:**

Für die Herstellung von Weichgelatinekapseln mit 5 mg Wirkstoff pro Kapsel eignet sich die folgende Zusammensetzung:

| | |
|---|---|
| Wirkstoff | 5 mg |
| Mischung von Triglyceriden aus Kokosöl | 150 mg |
| Kapselinhalt | 155 mg |

**Beispiel D:**

Für die Herstellung von Dragees eignet sich folgende Formulierung:

| | |
|---|---|
| Wirkstoff | 3 mg |
| Maisstärke | 100 mg |
| Lactose | 55 mg |
| sec. Calciumphosphat | 30 mg |
| lösliche Stärke | 3 mg |
| Magnesiumstearat | 5 mg |
| kolloidale Kieselsäure | 4 mg |
| | 200 mg |

**Beispiel E:**

Dragees, enthaltend einen erfindungsgemäßen Wirkstoff und einen anderen therapeutisch wirksamen Stoff:

| | |
|---|---|
| Wirkstoff | 6 mg |
| Propanolol | 40 mg |
| Milchzucker | 90 mg |
| Maisstärke | 90 mg |
| sec. Calciumphosphat | 34 mg |
| lösliche Stärke | 3 mg |
| Magnesiumstearat | 3 mg |

(fortgesetzt)

| | |
|---|---|
| kolloidale Kieselsäure | 4 mg |
| | 270 mg |

**Beispiel F:**

Dragees, enthaltend einen erfindungsgemäßen Wirkstoff und einen anderen therapeutisch wirksamen Stoff:

| | |
|---|---|
| Wirkstoff | 5 mg |
| Pirlindol | 5 mg |
| Milchzucker | 60 mg |
| Maisstärke | 90 mg |
| sec. Calciumphosphat | 30 mg |
| lösliche Stärke | 3 mg |
| Magnesiumstearat | 3 mg |
| kolloidale Kieselsäure | 4 mg |
| | 200 mg |

**Beispiel G:**

Kapseln, enthaltend einen erfindungsgemäßen Wirkstoff und einen anderen therapeutisch wirksamen Stoff:

| | |
|---|---|
| Wirkstoff | 5 mg |
| Nicergolin | 5 mg |
| Maisstärke | 185 mg |
| | 195 mg |

**Beispiel H:**

Injektionslösungen mit 1 mg Wirkstoff pro ml können nach folgender Rezeptur hergestellt werden:

| | |
|---|---|
| Wirkstoff | 1,0 mg |
| Polyethylenglykol 400 | 0,3 mg |
| Natriumchlorid | 2,7 mg |
| Wasser zu Injektionszwecken auf | 1 ml |

**Pharmakologische Daten:**

Geprüft wird die Hemmung der Bindung von Fibrinogen an seinen Rezeptor (Glykoprotein IIb/IIIa) an intakten, gelfiltrierten Human-Thrombozyten durch die erfindungsgemäßen Verbindungen. Angegeben ist der $K_i$-Wert der Bindungshemmung von $^{125}$I-Fibrinogen nach Stimulierung mit ADP (10µM).

Literatur:

J.S. Bennett u. G. Vilaire, J. Clin. Invest. 64 (1979), 1393-1401

E. Kornecki et al., J. Biol. Chem. 256 (1981), 5695-5701 G.A. Marguerie et al., J. Biol. Chem. 254 (1979), 5357-5363 G.A. Marguerie et al., J. Biol. Chem. 255 (1980), 154-161

| **Beispiel** | **$K_i$ (µM), ADP-stimuliert** |
|---|---|
| 4 | 0.32 |
| 7 | 0.17 |

Als funktioneller Test wird die Hemmung der Aggregation gelfiltrierter Human-Thrombozyten nach ADP- oder Thrombin-Stimuiierung durch die erfindungsgemäßen Verbindungen gemessen. Angegeben ist der $IC_{50}$-Wert der

Hemmung. Literatur:
G.A. Marguerie et al., J. Biol. Chem. 254 (1979), 5357-5363

| Beispiel | ADP-stimuliert | $IC_{50}$ (μM), Thrombin-stimuliert |
|---|---|---|
| 4 | 0.45 | 1.0 |
| 7 | 0.25 | 0.6 |

**Patentansprüche**

**1.** Verbindungen der allgemeinen Formel I

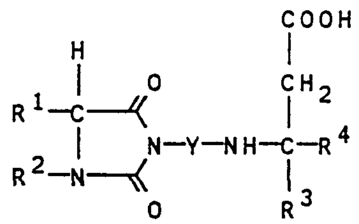

(I)

worin
Y $-(CH_2)_m-CO-$, wobei m für 1 oder 2 steht, oder

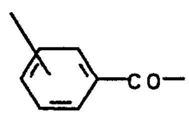

bedeutet;

$R^1$ $-CH_2-C_6H_4-NH-C(=NH)-NH_2$, $-CH_2-C_6H_4-C(=NH)-NH_2$ oder $-CH_2-C_6H_4-CH_2-NH_2$ bedeutet;
$R^2$ Wasserstoff oder Methyl bedeutet;
$R^3$ Wasserstoff bedeutet; und
$R^4$ $-CO-NH-R^5$ bedeutet, wobei $-NH-R^5$ für ein ω-Amino-($C_2$-$C_8$)-alkylamid des Valin-, Lysin-, Phenylalaninoder des Phenylglycinrestes steht;
sowie deren physiologisch verträgliche Salze.

**2.** Verbindungen gemäß Anspruch 1, **dadurch gekennzeichnet, daß** für $-NH-R^5$ das 4-Aminobutylamid des Valin-, Lysin-, Phenylalanin- oder des Phenylglycin-Restes steht.

**3.** Verfahren zur Herstellung von Verbindungen der Ansprüche 1 und 2, **dadurch gekennzeichnet, daß** man eine Fragmentkondensation einer Verbindung der allgemeinen Formel III

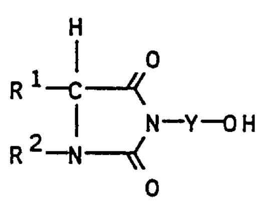

(III)

mit einer Verbindung der allgemeinen Formel IV

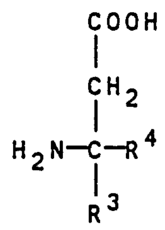

(IV)

wobei die Reste $R^1$ bis $R^4$ und Y wie in Anspruch 1 angegeben definiert sind, ausführt.

4. Verbindung der allgemeinen Formel I der Ansprüche 1 und 2 als Hemmstoff der Thrombozytenaggregation, der Metastasierung von Karzinomzellen oder der Osteoclastenbindung an die Knochenoberflächen.

5. Pharmazeutisches Präparat, **dadurch gekennzeichnet, daß** es eine oder mehrere Verbindungen der allgemeinen Formel I der Ansprüche 1 und 2 oder ein physiologisch verträgliches Salz davon als Wirkstoff zusammen mit pharmazeutisch annehmbaren Träger- und Zusatzstoffen und gegebenenfalls noch ein oder mehrere andere pharmakologische Wirkstoffe enthält.

6. Verfahren zur Herstellung eines pharmazeutischen Präparates, enthaltend eine oder mehrere Verbindungen der allgemeinen Formel I der Ansprüche 1 und 2 oder ein physiologisch verträgliches Salz davon, **dadurch gekennzeichnet, daß** man diese zusammen mit pharmazeutisch annehmbaren Träger- und Zusatzstoffen und gegebenenfalls noch ein oder mehreren anderen pharmakologischen Wirkstoffen in eine geeignete Darreichungsform bringt.

## Claims

1. Compounds of the general formula I

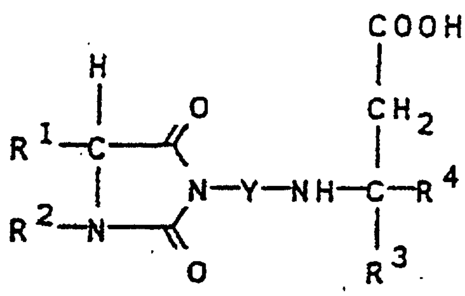

(I)

in which
Y denotes $-(CH_2)_m-CO-$, where m represents 1 or 2, or

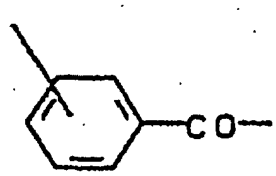

;

$R^1$ denotes $-CH_2-C_6H_4-NH-C(=NH)-NH_2$, $-CH_2-C_6H_4-C(=NH)-NH_2$ or $-CH_2-C_6H_4-CH_2-NH_2$;
$R^2$ denotes hydrogen or methyl;
$R^3$ denotes hydrogen; and

$R^4$ denotes -CO-NH-$R^5$, where -NH-$R^5$ represents an ω-amino-($C_2$-$C_8$)-alkyl amide of the valine, lysine, phenylalanine or phenylglycine residue;
and their physiologically tolerable salts.

2. Compounds according to Claim 1, **characterised in that** -NH-$R^5$ is represented by the 4-aminobutyl amide of the valine, lysine, phenylalanine or phenylglycine residue.

3. Process for the preparation of compounds of Claims 1 and 2, **characterised in that** a fragment condensation of a compound of the general formula III

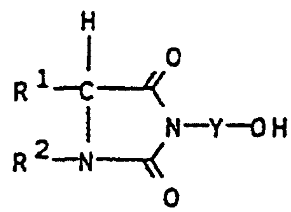

(III)

with a compound of the general formula IV

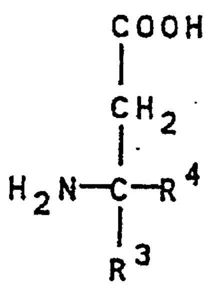

(IV)

where the radicals $R^1$ to $R^4$ and Y are defined as indicated in Claim 1, is carried out.

4. Compound of the general formula I of Claims 1 and 2 as an inhibitor of platelet aggregation, metastasis of carcinoma cells or osteoclast binding to the bone surfaces.

5. Pharmaceutical preparation, **characterised in that** it contains one or more compounds of the general formula I of Claims 1 and 2 or a physiologically tolerable salt thereof as active compound together with pharmaceutically acceptable excipients and additives and, if appropriate, also one or more other pharmacological active compounds.

6. Process for the production of a pharmaceutical preparation containing one or more compounds of the general formula I of Claims 1 and 2 or a physiologically tolerable salt thereof, **characterised in that** these are brought into a suitable administration form together with pharmaceutically acceptable excipients and additives and, if appropriate, also one or more other pharmacological active compounds.

**Revendications**

1. Composés de formule générale I :

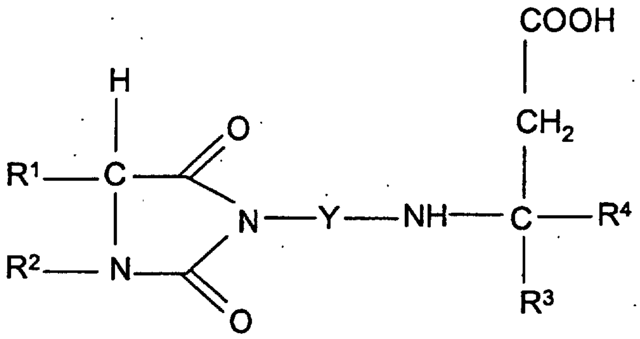

(I)

où

Y représente $-(CH_2)_m-CO-$, où m représente 1 ou 2, ou

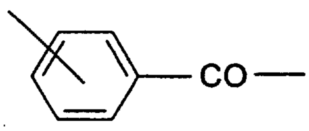

;

$R^1$ représente $-CH_2-C_6H_4-NH-C(=NH)-NH_2$, $-CH_2-C_6H_4-C-(=NH)-NH_2$ ou $-CH_2-C_6H_4-CH_2-NH_2$ ;
$R^2$ représente l'hydrogène ou méthyle ;
$R^3$ représente l'hydrogène ; et
$R^4$ représente $-CO-NH-R^5$ où $-NH-R^5$ représente un ω-amino-alkyle en $(C_2-C_8)$amide du reste valine, lysine, phénylalanine ou phénylglycine;
ainsi que leurs sels physiologiquement acceptables.

2. Composés selon la revendication 1, **caractérisés en ce que** $-NH-R^5$ représente le 4-aminobutylamide du reste vàline, lysine, phénylalanine ou phénylglycine.

3. Procédé de production de composés des revendications 1 et 2, **caractérisé en ce que** l'on conduit une condensation de fragments d'un composé de formule générale III

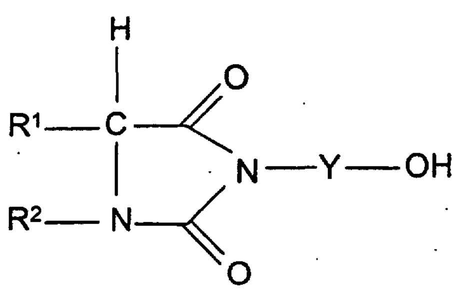

(III)

avec un composé de formule générale IV

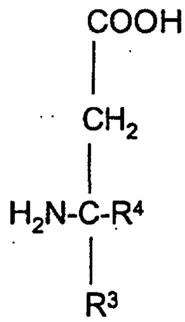

(IV)

où les restes $R^1$ à $R^4$ et Y sont définis de la manière indiquée dans la revendication 1.

**4.** Composé de formule générale I des revendications 1 et 2 comme inhibiteurs de l'agrégation des thrombocytes, de la formation de métastases de cellules cancéreuses ou de la liaison d'ostéoclastes aux surfaces des os.

**5.** Préparation pharmaceutique, **caractérisée en ce qu'**elle contient un ou plusieurs composés de formule générale I des revendications 1 et 2 ou un sel physiologiquement acceptable de ceux-ci comme principe actif ainsi que des supports ou additifs pharmaceutiquement acceptables et éventuellement encore un ou plusieurs autres principes actifs pharmacologiques.

**6.** Procédé de production d'une préparation pharmaceutique contenant un ou plusieurs composés de formule générale I des revendications 1 ou 2 ou un sel physiologiquement acceptable de ceux-ci, **caractérisé en ce qu'**on les met sous une forme d'administration appropriée en même temps que des supports et additifs pharmaceutiquement acceptables et éventuellement encore un ou plusieurs autres principes actifs pharmacologiques.